(19)

**Europäisches Patentamt**

**European Patent Office**

**Office européen des brevets**

(11) **EP 3 048 981 B1**

(12) **EUROPEAN PATENT SPECIFICATION**

(45) Date of publication and mention
of the grant of the patent:
**02.08.2017 Bulletin 2017/31**

(51) Int Cl.:
*A61B 8/12* (2006.01)    *A61B 8/08* (2006.01)
*G06F 19/00* (2011.01)    *G06T 7/00* (2017.01)

(21) Application number: **14796545.3**

(22) Date of filing: **23.09.2014**

(86) International application number:
**PCT/IB2014/001915**

(87) International publication number:
**WO 2015/044751 (02.04.2015 Gazette 2015/13)**

(54) **APPARATUS AND METHOD FOR PROCESSING CLINICAL AND BIOCHEMICAL DATA AND THREE-DIMENSIONAL ULTRASOUND IMAGES FOR PREDICTING OVARIAN AGE OF THE WOMAN, AND CORRESPONDING DIAGNOSTIC ULTRASOUND EQUIPMENT**

VORRICHTUNG UND VERFAHREN ZUR VERARBEITUNG KLINISCHER UND BIOCHEMISCHER DATEN UND DREIDIMENSIONALER ULTRASCHALLBILDER ZUR VORHERSAGE DES EISPRUNGALTERS EINER FRAU SOWIE ENTSPRECHENDE DIAGNOSTISCHE ULTRASCHALLVORRICHTUNG

APPAREIL ET PROCÉDÉ POUR TRAITER DES DONNÉES CLINIQUES ET BIOCHIMIQUES ET DES IMAGES ULTRASONORES TRIDIMENSIONNELLES POUR PRÉDIRE L'ÂGE OVARIEN DE LA FEMME, ET ÉQUIPEMENT ULTRASONORE DE DIAGNOSTIC CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2013 IT CZ20130017**

(43) Date of publication of application:
**03.08.2016 Bulletin 2016/31**

(73) Proprietor: **Ovage S.r.l.**
**88056 Tiriolo (CZ) (IT)**

(72) Inventors:
• **LICO, Daniela**
**89121 Reggio Calabria (RC) (IT)**
• **VENTURELLA, Roberta**
**88040 Settingiano (CZ) (IT)**
• **SARICA, Alessia**
**88100 Catanzaro (CZ) (IT)**

(74) Representative: **Scorza, Federica**
**Studio Rubino Srl**
**Via L. della Valle, 84**
**88100 Catanzaro (IT)**

(56) References cited:
• **MARIA ELISABETTA COCCIA ET AL: "Ovarian Reserve", ANNALS OF THE NEW YORK ACADEMY OF SCIENCES, vol. 1127, no. 1, 2 April 2008 (2008-04-02), pages 27-30, XP055118135, ISSN: 0077-8923, DOI: 10.1196/annals.1434.011**
• **JAYAPRAKASAN K ET AL: "Establishing the intercycle variability of three-dimensional ultrasonographic predictors of ovarian reserve", FERTILITY AND STERILITY, ELSEVIER SCIENCE INC, NEW YORK, NY, USA, vol. 90, no. 6, 2 December 2008 (2008-12-02), pages 2126-2132, XP025711103, ISSN: 0015-0282, DOI: 10.1016/J.FERTNSTERT.2007.10.028 [retrieved on 2008-01-14]**
• **YOUNIS J S ET AL: "A simple multivariate score could predict ovarian reserve, as well as pregnancy rate, in infertile women", FERTILITY AND STERILITY, ELSEVIER SCIENCE INC, NEW YORK, NY, USA, vol. 94, no. 2, 2 July 2010 (2010-07-02), pages 655-661, XP027104877, ISSN: 0015-0282 [retrieved on 2009-04-14]**

EP 3 048 981 B1

**Description**

"Apparatus and method for processing clinical and biochemical data and three-dimensional ultrasound images for predicting ovarian age of the woman, and corresponding diagnostic ultrasound equipment"

The present invention relates to an apparatus and method for processing clinical and biochemical data and three-dimensional ultrasound images for predicting ovarian age of the woman, and corresponding diagnostic ultrasound equipment.

As is well known, the ovarian age, or ovarian reserve, is defined as the follicular pool remaining in the woman ovaries at a particular time of her life. In particular, the ovarian reserve describes two aspects closely related to each other: the quantity and the quality of oocytes. The oocytes are formed only during fetal life and at the 20th week of intrauterine life the follicular pool is already fully formed. Since that time, the follicles begin a process of inexorable and progressive depletion. At birth, many oocytes have already undergone to apoptosis, or cell death, and the primordial follicles are present in numbers comprised between 700,000 and 1 million. This number represents the ovarian reserve of every woman, at birth. Physiologically there is a gradual reduction in the number of primordial follicles due to degenerative phenomena that determine the atresia. In adult women, the rate of consumption of follicles is not constant, but accelerates with age. Therefore, only 12% of the assets follicular present at birth remains in the ovary already when the women are 30 years old, and only 3% when the women are 40 years old.

It's also known, however, that the normal aging process of reproduction varies greatly among women. Some women, in fact, maintain fertility in long, even up to the fifth decade of their life, while others often lose the natural fertility already in the middle of the third decade of their life. The individual variability depends on the age of onset of menopause, normally at 51 years old, but possibly between 40 and 60 years of the women. About 10% of women eventually reach menopause by the age of 45 years.

Over the past two decades, the possibility of a precise prediction of the age of onset of menopause has aroused much interest. Nowadays, many women postpone their pregnancy due to social and economic reasons, with considerable anxiety and uncertainty about the amount of time left before reaching the menopause. The prediction of age of the menopause arriving could certainly help these women to make a reasonable decision regarding their family planning. Still, from the gynecological point of view, to know the age of onset of menopause has important clinical implications in terms of surgical indications and well-being of women. In fact, there are many benign gynecological diseases for which the menopause is a natural cure, such as an example in the case of women having uterine fibroids, the quality of their life being often severely deteriorated due to abnormal uterine bleeding and extremely abundant menstrual flows often causing anemia. For all these women, menopause is the time of their reproductive life in which the symptoms cease naturally. However, because it's not possible to predict the arrival of this phase, today almost all of the patients undergoes surgery demolition, including the removal of the uterus, with a remarkable economic impact on the National Health Service and, not least, on the life of the woman herself.

Several attempts have been made so far to predict the age of menopause and to assess the status of ovarian reserve. Despite the subtle changes in the endocrine regulation of ovarian function with advancing age, however, the majority of individual indicators studied so far not proved to be clinically useful for the prediction of menopause. The most important of these indicators is the chronological aging which is related to the reproductive aging in relation to changes in ovarian function. The reduction in the number of follicles coincides with a reduction in the quality of the oocytes, due to the gradual change in the regularity of the menstrual cycle and of the monthly fertility, inevitably evolving to menopause over time. In addition, to the normal female reproductive aging, there are large individual variations in the age of occurrence of variations in reproductive events. Although chronological age is the most important factor to predict the physiological response of the ovary to the exogenous and endogenous action of the follicle stimulating hormone (FSH), both environmental factors and genetic factors contribute to biological aging of the ovary, and, for this reason, very often the chronological age and the biological age are not equivalent. Among the genetic factors, the ones that allows to determine at what age the menopause will arrive, the most important are the size of the initial set of oocytes, the proportion of the oocytes undergoing atresia, or programmed cell death, and the rate of initiation of follicular growth. This has also suggested that some polymorphisms of some single nucleotides influence receptivity to gonadotropins and ovarian aging. In particular, environmental factors such as diet, cigarette smoking, chemotherapy or radiation therapy regimens, surgery of the endometrium or of the ovary may also make shorter the life of the ovary of a woman. Although the continued loss of follicles could be recognized only at an advanced stage by the irregularity of the cycle, endocrine and ultrasonographic markers have been found over the last decade, which could express more accurately the decline of the number of follicles in a long period of time. Among the various endocrinological parameters, the most widely studied and used in clinical practice are the Follicle Stimulating Hormone (FSH), the estradiol (E2), and the Antimulleriano Hormone (AMH).

In particular, the basal FSH, studied a lot over the last few years, is the more indirect marker of ovarian reserve. FSH levels increase with age, due to the reduction of the negative feedback on the release of FSH from the pituitary. To exclude the possibility that confounding factors, such as levels of E2 higher than 50pg/ml which suppresses the secretion of FSH, can occur, these parameters must always be evaluated together between the first and the fourth day of the cycle.

In women with varying degrees of ipergonadotropysm, however, the best parameter to assess the extent of the depletion of ovarian follicles is represented by AMH. It is a dimeric glycoprotein with autocrine and paracrine action in the developing follicles, exclusively produced by granular cells of preantral follicles and of the small antral follicles (2-7 mm in diameter). The number of small antral follicles is directly correlated with the total quantity of primordial follicles. With the decrease in the number of antral follicles with age, also the serum level of AMH decreases and inevitably becomes not measurable when the menopause is arriving. Recent studies have shown that serum levels of AMH represent a quantitative assessment of ovarian reserve and may provide an index of the age at which menopause arrives.

Regarding the ultrasonographic markers, several studies have recently shown that the counting of primordial follicles, defined as the total number of antral follicles (AFC) having the size of 2-10 mm in diameter and present in both ovaries, represents a best marker of both chronological age and the basal levels of FSH, to evaluate the biological age of the ovary. As it is for AFC, the ovarian volume, measured by means of transvaginal ultrasound, has been associated with ovarian function, and also the vaginal ultrasonography using the "power Doppler" is often used to assess the blood flow through the ovaries in the natural cycle.

A first known solution is known from the paper "Ovarian reserve", of MARIA ELISABETTA COCCIA ET AL: ANNALS OF THE NEW YORK ACADEMY OF SCIENCES; vol.1127, no. 1, 1 April 2008, pages 27-30, XP055118135. This paper describe that as a result of temporary social trends, many women elect to postpone their first pregnancy to a later stage in life. A large part of this population will be infertile by the time they opt to conceive, mainly because of a decreasing ovarian reserve and low oocyte quality resulting from age. Aging oocytes have been widely suggested to be the major cause for the decline in fertility. In a subfertile population, the availability of an accurate screening test of ovarian reserve would provide a valuable means of predicting the chances of pregnancy and live birth with or without treatment and selecting an optimal dose of ovarian stimulation where treatment using ovarian stimulation is planned. The following hormonal markers and ultrasound parameters have been used to attempt to estimate ovarian reserve and predict those with a poor chance of success in assisted reproductive techniques: age; concentrations of follicle-stimulating hormone (FSH), luteinizing hormone (LH), estradiol, inhibin, anti-Müllerian hormone; ovarian volume, ovarian antral follicle count; and ovarian biopsy. Further studies have introduced the use of dynamic tests-using gonadotropin-releasing hormone agonist, FSH, or clomiphene citrate-to assess ovarian function. The use of a wide range of tests suggests that no single test provides a sufficiently accurate result. But the simultaneous evaluation of a combination of tests could be used as a marker of diminished ovarian reserve and a sensitive predictor of response to ovarian stimulation in patients undergoing *in vitro* fertilization treatment.

A second solution is known from the paper "Establishing the intercycle variability of three-dimensional ultrasonographic predictors of ovarian reserve", of JAYAPRAKASAN K ET AL: FERTILITY AND STERILITY, ELSEVIER SCIENCE INC, NEW YORK, NY, USA, vol. 90, no. 6, 1 December 2008, pages 2126-2132, XP025711103. The paper describes how to estimate the intercycle variability of antral follicle counts (AFCs) and ovarian volume, as measured by using three-dimensional ultrasound, and to compare these to the variation in basal FSH levels. The solution uses transvaginal three-dimensional ultrasound assessment and venepuncture in the early follicular phase of the menstrual cycle, immediately before assisted reproductive technology. The main outcome are intercycle variability of AFC, ovarian volume, and basal FSH and the conclusion is that the AFC demonstrates a lower intercycle variability than do ovarian volume and basal FSH level. The observed intercycle variability of the AFC may primarily be caused by observer variability, and the true biological variation may be minimal.

A third solution is known form the paper "A simple multivariate score could predict ovarian reserve, as well as pregnancy rate, in infertile women", of YOUNIS J S ET AL., FERTILITY AND STERILITY, ELSEVIER SCIENCE INC, NEW YORK, NY, USA, vol. 94, no. 2, 1 July 2010, pages 655-661, XP027104877. The scope is to find a simple multivariate score that has the potential to predict ovarian reserve, as well as pregnancy rate, in infertile women. Basal ovarian reserve studies, endocrine and sonographic, were performed before starting therapy. After completion of treatment, a logistic regression analysis was performed to examine which parameters significantly determined low ovarian reserve. These parameters were incorporated thereafter in a multivariate score to predict ovarian reserve, as well as clinical pregnancy rate. Main Outcome is low ovarian reserve defined as%3 oocytes on retrieval day and clinical implantation and pregnancy rates. The paper describes that a novel and simple multivariate score using clinical and basal endocrine and sonographic parameters has a distinctive prediction of low ovarian reserve in infertile women undergoing assisted reproductive technology treatment. Moreover, it has the potential to predict clinical implantation and pregnancy rates in women with low and good ovarian reserve.

However, the methods used so far to predict the woman's ovarian age have the problem of making measurements of the individual endocrinological and ultrasonographic parameters described above. Therefore, since these parameters are considered markers with low specificity and sensitivity when they are used to predict ovarian reserve in infertile women and to prognosticate the arriving of the menopause, such methods do not provide a precise and accurate answer of the ovarian age.

Purpose of the present invention is to provide an apparatus and method for processing clinical and biochemical data and three-dimensional ultrasound images for predicting ovarian age of the woman, and corresponding diagnostic ultrasound equipment, having characteristics such as to overcome the limitations which still affect the apparatuses and methods previously described with reference to the known technique.

According to the present invention, an apparatus for processing clinical and biochemical data and ultrasound three-dimensional images for predicting ovarian age of the woman is realized, as defined in claim 1.

Furthermore, according to the present invention, a method of processing clinical and biochemical data and ultrasound three-dimensional images for predicting ovarian age of the woman is realized, as defined in claim 2.

Additionally, according to the present invention a diagnostic ultrasound equipment for processing clinical and biochemical data and ultrasound three-dimensional images for predicting ovarian age of the woman is realized, as defined in claim 3. For a better understanding of the present invention a preferred embodiment is now described, purely as a nonlimiting example, with reference to the accompanying drawings, in which:

- Figure 1 shows a schematic diagram of an apparatus for processing clinical and biochemical data and ultrasound three-dimensional images for predicting ovarian age of the woman, according to the invention;
- Figure 2 shows a flow diagram of a method for processing clinical and biochemical data and ultrasound three-dimensional images for predicting ovarian age of the woman, according to the invention;

- Figure 3 shows a flow diagram of a first embodiment, in the case of study carried out on a number N of patients, of the method for processing clinical and biochemical data and ultrasound three-dimensional images for predicting ovarian age of the woman, according to the invention;
- Figure 4 shows a flow diagram of a second embodiment of the method for processing clinical and biochemical data and ultrasound three-dimensional images for predicting ovarian age of the woman, according to the invention.

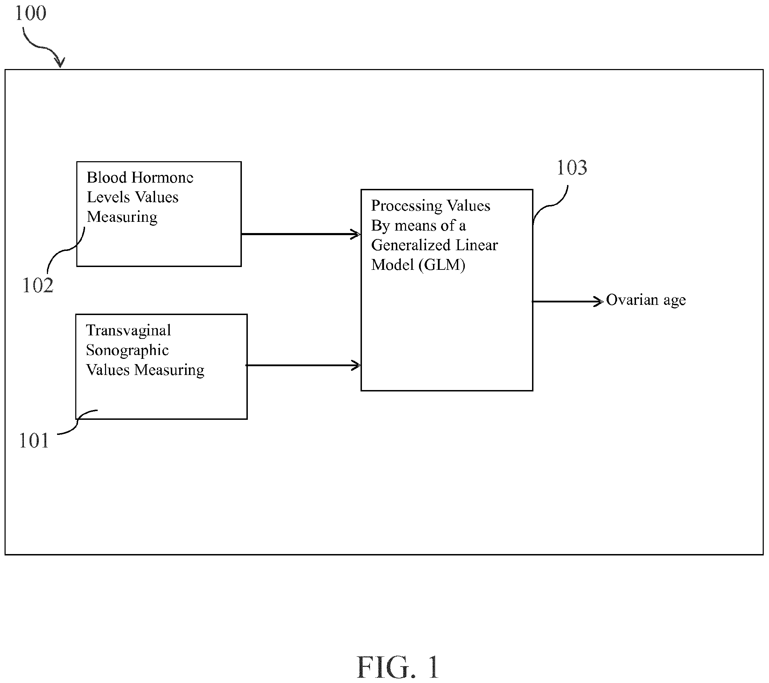

With reference to these figures, and, in particular, to Figure 1, an apparatus 100 for processing clinical and biochemical data and ultrasound three-dimensional images for predicting ovarian age of the woman is shown, according to the invention. In details, the apparatus 100 for processing clinical and biochemical data and ultrasound three-dimensional images for predicting ovarian age of the woman comprises a function block 101 for measuring transvaginal sonographic values and a function block 102 for measuring blood hormone levels having the output parameters ready to be processed by a block 103 for processing data that uses a processing generalized linear model (GzLM) and which provides, as output, the ovarian age measured in years. More in detail, as an example, the functional block 101 is configured to measure trans-vaginal ultrasound values of the ovarian volume, of the antral follicle count (AFC), of the Index of vascularization (VI), of the flow rate (FI), of the index of vascular flow (VFI) of a woman. Instead, the functional block 102 is configured for measuring blood hormonal values of at least one woman comprised in the group constituted by Antimullerian Hormone (AMH), Follicle Stimulating Hormone (FSH), Estradiol (E2), in healthy women during a period of time comprised between the first and fourth day of the menstrual cycle.

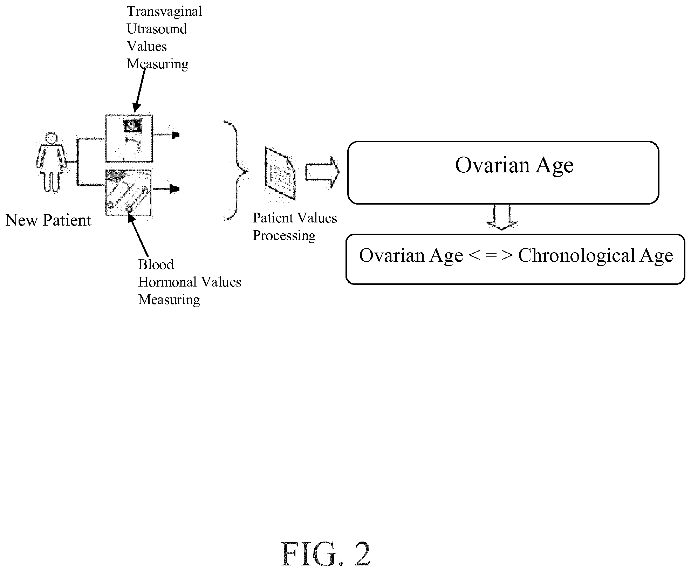

As already explained, the present application also relates to a method for processing clinic and biochemical data and three-dimensional ultrasound images for predicting the ovarian age of the woman. More in details, as shown in the flowchart of Figure 2, the method comprises the steps of:

- Measuring, by a simple blood sample of a patient, the blood hormonal values, for example the basal levels of AMH, FSH, estradiol or E2, in healthy women over a time period comprised between the first and the fourth day of the menstrual cycle;
- Measuring, by means of 3D ultrasound on the patient, transvaginal ultrasound values, such as the ovarian volume, the counting of antral follicles (AFC), the Index of Vascularization (VI), the flow rate (FI) and the index of vascular flow (VFI);

- Performing a processing of the blood hormonal parameters and of transvaginal ultrasound parameters using a polynomial function configured for combining the parameters Antimullerian Hormone (AMH), Follicle Stimulating Hormone (FSH), Estradiol (E2), the total number of antral follicles (AFC), the Flow Index (FI), the Index of Vascularization (VI), the index of vascular flow (VFI) and the ovarian volume;
- Outputting the value of ovarian age;
- Comparing the value of the ovarian age with the value of the chronological age of the woman.

More specifically, blood samples are evaluated according to standard methods, while the ultrasound parameters are obtained by 3D ultrasound acquisition, shown in multiplanar prospects in the echography equipment. The displayed image is optimized and the rendering mode generates the interested three-dimensional volume.

Advantageously according to the invention, the render box is adjusted to exclude as much as possible extra-ovarian information and to ensure that the entire ovary is included in the ovarian volume, VOI.

According to an aspect of the invention, the set of ultrasound parameters is examined using a software, such as SonoAVC, which identifies and quantifies hypo-echoic regions within the 3D ultrasound dataset and provides an automatic estimation of their extent. A specific color is assigned to each individual volume, and the automatic measurement of its major diameters and of its maximum size (x, y, x) are shown in descending order, from the largest one to the smallest one. The individual follicles are shown with a specific color and shown together with their size and relative measures. In addition, an operation of post-processing involving the manual identification of the follicles, not automatically included in the analysis, is implemented to ensure that all antral follicles are counted and measured.

Advantageously according to the invention, both ovaries are scanned with the power Doppler.

According to an aspect of the invention, the built-in Vocal (virtual organ computer-aided analysis) Imaging Program for 3D power Doppler is used to determine the ovarian volume, the indices of vascularization and the flow rate index. The index of vascularization VI measures the number of color of the voxels that represent the blood vessels in the ovary and is expressed as a percentage (%) of the ovarian volume. Instead, the Flow Index (FI) incorporates the color value of the voxels and represents the average intensity of the flow inside the ovary. The parameter VFI is obtained by multiplying the parameters VI and FI and is the combination of vascularization and flow quantity.

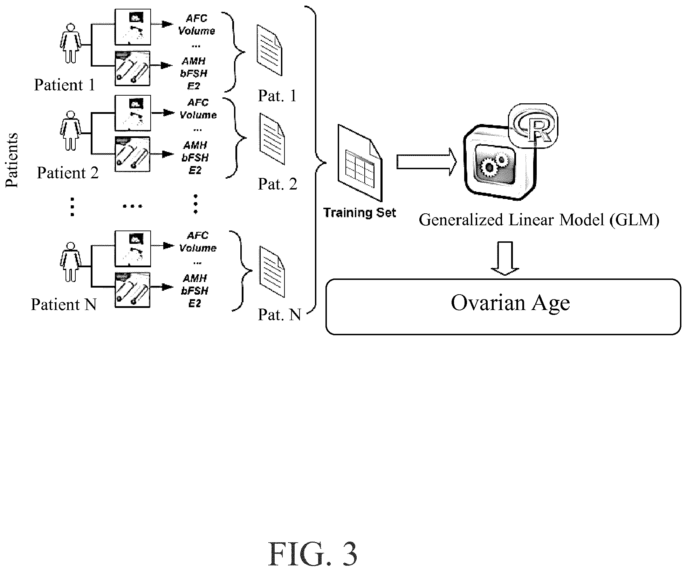

Advantageously according to the invention, as shown in Figure 3, the data of a plurality of patients may be extracted, so as to collect a number N of data records, the so-called Training Set, available to be processed by a Generalized Linear Model GzLM, able to provide the value of the ovarian age. More in particular, a statistic operation is performed. Specifically, the values of 8 parameters (AMH, bFSH, E2, AFC, FI, VI, VFI, Volume) are examined for all patients, for which the analysis of descriptive statistics is performed, including chronological age.

The Applicants have conducted experimental tests of the method for processing clinical and biochemical data and three-dimensional ultrasound images for predicting ovarian age of the woman. In particular, by means of histograms, normality Q-Q graphs and box-plots of each variable, the non-normality of some distributions are carried out. To obtain a quantitative determination of the non-normality of the distributions, the Applicants have used the two most common tests, the Kolmogorov-Smirnov test and the Shapiro-Wilk test. To calculate the correlation between two variables the correlation coefficient between Spearman's ranks (Spearman's Rho) has been used. It is a nonparametric correlation measure that assumes a monotonic arbitrary function to describe the relationship between two variables, without making any assumptions about the frequency distribution of the variables. The evaluation tests shown that: 1) In all three tests, there is a strong negative correlation between age and AMH levels: the values of AMH decrease with increasing age. 2) In all three tests, there is a strong positive correlation between age and levels of bFSH: the bFSH levels values increase with increasing age. 3) In all three tests, there is a strong negative correlation between age and levels of E2: the levels values of E2 decrease with increasing age. 4) In all three tests, there is a moderate negative correlation between age and levels of AFC: the levels values of AFC decrease with increasing age, although not strongly. 5) In all three tests, there is a moderate negative correlation between age and levels of FI: the levels values of FI decrease with increasing age, although not strongly. 6) In all three tests, there is a strong negative correlation between age and levels of Volume: the levels values of volume decrease with increasing age. 7) Two of the three tests (Pearson and Spearman) assess that there is no significant correlation between age and the value of VFI. The evaluation of the quality of the model was conducted using the concept of power developed by Cohen (1988), as well as by means of deviance and AIC parameter. According to the Cohen formula, for a determined significance level equal to 0.05, for an effect size equal to 0.02, for a fixed number of patients and predictors, a power equal to 0.80 was obtained. This result was obtained through a preliminary study of the sample size, i.e. the required number of individuals, entities or samples to observe and study, which is a crucial component of the design phase of any laboratory or clinic investigation.

Therefore, a generalized linear model (GzLM) was created to model the response of the dependent variables that do not have a normal distribution, which is instead the premise of the linear regression. The GzLM model is also suitable for a situation in which the link between the independent variable and the dependent variables is not linear, and in the case of error variance not constant. The parameters of the GzLM are estimated by the method of maximum likelihood (ML). The logistic regression (when the response variable is dichotomic type and binomial) and the Poisson regression (when the response is a counting variable with Poisson distribution) can be treated using the GzLM.

The cases with suspected premature menopause were eliminated from the initial dataset of healthy patients. RCommander was used for the generation of the GzLM model. In fact, RCommander is a graphical interface R programming language which allows to assess iteratively (stepwise) the goodness of the model and consequently to eliminate the features with low predictive power. The output from the generation of the model are the coefficients and the intercept of the polynomial equation, which in general is called the regression equation. The generated equation is the following:

```
Ovarian age = c + a*AMH + b*bFSH + c*E2 + d*AFC + e*FI
   + f*VFI + g*Volume.
```

Advantageously according to the invention, the algorithm excludes the not explanatory curves, i.e. the curves which have a p value > 0.05. In particular, the generalized linear model comprises a linear predictor, which is the systematic component, and a random component, i.e. independent Gaussian random variables that model the error. The so-called "link function" combines these two components and, having assumed the chronological age as a variable having the Poisson distribution, the chosen link function is logarithmic.

Advantageously according to the invention, the deviance of the residues and the Aikake Information Criterion (AIC) are evaluated. These two metrics allow to compare different models with different characteristics. Using graphical representation of Cook's distances for each sample of the "Training set" all those cases (levers) that adversely affect the power of prediction are eliminated. Refinement of the model ends when the average of the difference between chronological age and ovarian age of the patients is approximately ± 2 years. The value of this difference is clinically acceptable for the purposes of predicting ovarian age.

At this point, the predictions for the previously excluded patients (with suspected premature menopause) are generated.

The Applicant have proved that for all cases the predicted age was higher than chronological age.

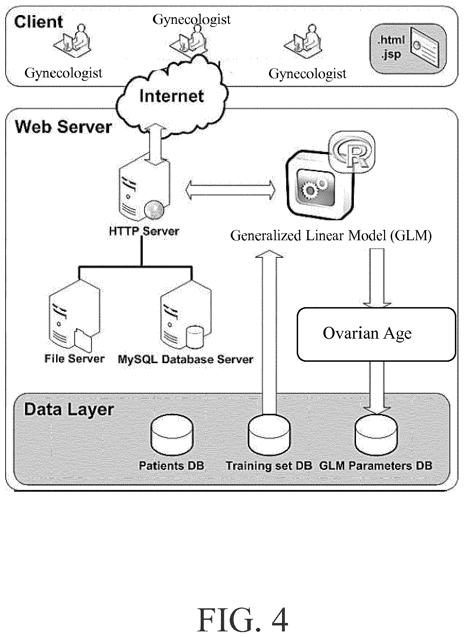

According to an aspect of the invention, the described method can be implemented as shown in Figure 4, by means of a web-based software, so that each gynecologist, connecting to the personal Internet page and making the login in using own access credentials can provide the patient's blood and ultrasound values as an input to a HHTP server, which leads to a File server and to a MySQL Database server. The Generalized Linear Model runs on the server using the data Training Set on the server, performs its processing and appropriate comparisons and obtains, as a result of the polynomial equation, the real-time ovarian age. In this way, the server that generates the response acquires the data of the new patient, increasing the data set of the model and improving time to time the predictive accuracy of the method.

According to another aspect of the invention, the apparatus described can be implemented on ecograph using a 3D software, so that, once performed the ultrasound examination, it's possible to enter as input the only biochemical data and the ecograph will be able to output the ovarian age and the reference curves of the general population.

Therefore, the apparatus and the method for processing clinical and biochemical data and three-dimensional ultrasound images for predicting ovarian age of the woman according to the invention allow to overcome the problems related to the intra and inter individual variation of the characteristics assessed from time to time, respond to the request of the gynecologist and of the woman providing an objective, numeric and therefore intuitive result.

Another advantage of the apparatus and the method for processing clinical and biochemical data and three-dimensional ultrasound images for predicting ovarian age of the woman according to the invention consists in the fact that the gynecologist can use an innovative, original, easy to use and fast response tool helpful to guide the most appropriate treatment decisions for the patients, helping the young women who have not yet had to pregnancies to safely plan their reproductive desire, consciously deciding how much to anticipate or postpone the search of motherhood.

A further advantage of the apparatus and the method for processing clinical and biochemical data and three-dimensional ultrasound images for predicting ovarian age of the woman according to the invention consists in the fact that allow to avoid unnecessary and possibly harmful surgeries in old women, approaching menopause, for which it will be possible to indicate the remaining time to the end of menstruation. The unnecessary hysterectomies, performed unconsciously close to the natural menopause, will be reduced.

A further advantage of the apparatus and the method for processing clinical and biochemical data and three-dimensional ultrasound images for predicting ovarian age of the woman according to the invention is that it allows to schedule a surgery for conditions such as endometriosis or myomas, close to the desire of the reproductive patients, with the right timing and in accordance with the ovarian age, rather than with the chronological age of the patient, thus reducing the use of second and third surgery, that often lead to surgical complications and reproductive disasters.

Another advantage of the apparatus and the method for processing clinical and biochemical data and three-dimensional ultrasound images for predicting ovarian age of the woman according to the invention consists in the fact that the gynecologist will be able to quantify the influence of particular diseases, which have a negative effect on ovarian reserve, or of gynecological surgeries that need to involve the removal of healthy ovarian tissue.

Another advantage of the apparatus and the method for processing clinical and biochemical data and three-dimensional ultrasound images for predicting ovarian age of the woman according to the invention consists in the fact that the gynecologist will be able to drive the choices of the pairs with fertility problems, indicating the most appropriate reproductive method and avoiding costly over-treatment and eliminating the delays in treatment for techniques of in vitro fertilization.

A further advantage of the apparatus and the method for processing clinical and biochemical data and three-dimensional ultrasound images for predicting ovarian age of the woman according to the invention consists in the fact that the gynecologist will be able to precisely identify the starting dose of the drugs used for controlled ovarian stimulation in women undergoing assisted reproduction cycles by reducing complications such as the lack of response in the event of insufficient dose, resulting in a lengthening of the time and amount of drug use (increased costs) and possible reduction in the quality of the response in terms of oocyte quality at the time of sampling. Instead, in this way, it's possible to avoid that the couples live the experience of a painful failure, with considerable economic and psychological implications, and to avoid to give the woman a starting dose higher than that required thus reducing the risk of medical complications.

Finally, the apparatus and the method for processing clinical and biochemical data and three-dimensional ultrasound images for predicting ovarian age of the woman according to the invention is non-invasive, requiring the execution of a simple blood sampling at a given time of the menstrual cycle, and a trans-vaginal ultrasound evaluation, both routine methods for the gynecologist and for women in reproductive age.

Finally it is clear that the apparatus and the method for processing clinical and biochemical data and three-dimensional ultrasound images for predicting ovarian age of the woman described and illustrated here can be modified and varied without departing from the protection scope of the present invention, as defined in the appended claims.

## Claims

1.  Apparatus (100) for processing clinical and biochemical data and ultrasound three-dimensional (3D) images for the prediction of the woman ovarian age, comprising:

    - at least one functional block (101) configured for measuring trans-vaginal ultrasound values of the ovarian volume, of the antral follicle count (AFC), of the Index of vascularization (VI), of the flow rate (FI), of the index of vascular flow (VFI) of at least one woman;
    - and at least one functional block (102) configured for measuring blood hormonal values of at least one woman comprised in the group constituted by Antimullerian Hormone (AMH), Follicle Stimulating Hormone (FSH), Estradiol (E2), in healthy women during a period of time comprised between the first and fourth day of the menstrual cycle;

    the output signals from said blocks (101, 102) being the input signals of a data processing block (103) based on a processing Generalized Linear Model (GzLM) constituted by a polynomial function configured for:

    - combining the parameters Antimullerian Hormone (AMH), Follicle Stimulating Hormone (FSH), Estradiol (E2), Antral follicle count (AFC), the Flow Index (FI), the Index of vascularization (VI), the index of vascular flow (VFI) and the ovarian volume, and
    - providing as the output result of the polynomial algorithm the value of the ovarian age as a function of the blood hormonal values and of the trans-vaginal ultrasound values, measured in years and compared to the woman age; wherein the polynomial function corresponds to the equation:

    ```
    ovarian age = c + a*AMH + b*bFSH +c*E2 + d*AFC + e*FI
    + f*VFI + g*Volume.
    ```

2.  Method for processing clinical and biochemical data and ultrasound three-dimensional (3D) images for the prediction of the woman ovarian age, comprising the steps of:

    - Measuring, through a simple blood test, blood hormonal values of at least one woman comprised in the group constituted by Antimullerian Hormone (AMH), Follicle Stimulating Hormone (FSH), Estradiol (E2), in healthy women during a period of time comprised between the first and fourth day of the menstrual cycle;
    - Measuring, through an ultrasound 3D echography, trans-vaginal ultrasound values of the ovarian volume, of the antral follicle count (AFC), of the Index of vascularization (VI), of the flow rate (FI), of the index of vascular flow (VFI) of at least one woman;
    - Performing the data processing of the blood hormonal and of the trans-vaginal ultrasound values by means of a processing generalized linear model (GzLM) constituted by a polynomial function configured for combining the parameters Antimullerian Hormone (AMH), Follicle Stimulating Hormone (FSH), Estradiol (E2), Antral follicle count (AFC), the Flow Index (FI), the Index of vascularization (VI), the index of vascular flow (VFI) and the ovarian volume;
    - providing, as an output value of the polynomial algorithm, the woman ovarian age as a function of the blood hormonal and trans-vaginal ultrasound values;
    - Comparing the woman ovarian age value with the woman age value; wherein the step of providing, as an output value of the polynomial algorithm, the woman ovarian age as a function of the blood hormonal and trans-vaginal ultrasound values comprises using the equation:

```
ovarian age = c + a*AMH + b*bFSH +c*E2 + d*AFC + e*FI
+ f*VFI + g*Volume.
```

3. Ultrasonic diagnostic apparatus for processing clinical and biochemical data and ultrasound three-dimensional (3D) images for the prediction of the woman ovarian age according, **characterized in** comprising the apparatus according to claim 1.

4. Processing program for processing clinical and biochemical data and ultrasound three-dimensional (3D) images for the prediction of the woman ovarian age, **characterized in** implementing the method according to claim 2 on a "web-based" software able to provide the woman ovarian age in real time.

**Patentansprüche**

1. Vorrichtung (100) für die Verarbeitung von klinischen und biochemischen Daten und dreidimensionalen (3D) Ultraschallbildern zur Vorhersage des Eierstockalters einer Frau, umfassend:

- mindestens einen Funktionsblock (101), konfiguriert für die Messung transvaginaler Ultraschallwerte des Ovarialvolumens, der Antralfollikelzählung (AFC), des Vaskularisationsindex (VI), der Flussrate (FI), des vaskulären flussindex (VFI) von mindestens einer Frau;
- und mindestens einen Funktionsblock (102), konfiguriert für die Messung von Blut-Hormonwerten mindestens einer Frau, die zur Gruppe bestehend aus Anti-Müller-Hormon (AMH), follikelstimulierendem Hormon (FSH) und Estradiol (E2) von gesunden Frauen in einem Zeitraum zwischen dem ersten und vierten Tag des Menstruationszyklus gehören;

wobei die Ausgangssignale der Blöcke (101, 102) die Eingangssignale eines Datenverarbeitungsblocks (103) sind und auf einem generalisierten linearen Verarbeitungsmodell (GzLM) basieren, das von einer Polynomfunktion gebildet wird und konfiguriert ist für:

- die Kombination der Parameter des Anti-Müller-Hormons (AMH), des follikelstimulierenden Hormons (FSH), Estradiols (E2), der Antralfollikelzählung (AFC), des Flussindex (FI), des Vaskularisationsindex (VI), des vaskulären Flussindex (VFI) und des Ovarialvolumens, und
- das Bereitstellen des Werts des Eierstockalters als eine Funktion der Blut-Hormonwerte und der transvaginalen Ultraschallwerte, die in Jahren gemessen und mit dem Alter der Frau verglichen werden, als Ausgangsergebnis des Polynomalgorithmus;

wobei die Polynomfunktion der folgenden Gleichung entspricht:

```
Eierstockalter = c + a*AMH + b*bFSH + c*E2 + d*AFC +
e*FI + f*VFI + g*Volumen.
```

2. Verfahren für die Verarbeitung von klinischen und biochemischen Daten und dreidimensionalen (3D) Ultraschallbildern zur Vorhersage des Eierstockalters einer Frau, umfassend die folgenden Schritte:

- Messung mithilfe eines einfachen Bluttests der Blut-Hormonwerte mindestens einer Frau, die zur Gruppe bestehend aus Anti-Müller-Hormon (AMH), follikelstimulierendem Hormon (FSH) und Estradiol (E2) bei gesunden Frauen in einem Zeitraum zwischen dem ersten und vierten Tag des Menstruationszyklus gehören;
- Messung mithilfe einer 3D-Ultraschall-Echografie der transvaginalen Ultraschallwerte des Ovarialvolumens, der Antralfollikelzählung (AFC), des Vaskularisationsindex (VI), der Flussrate (FI), des vaskulären Flussindex (VFI) von mindestens einer Frau;
- Durchführung der Datenverarbeitung der Bluthormon- und transvaginalen Ultraschallwerte mittels eines generalisierten linearen Verarbeitungsmodells (GzLM), das durch eine Polynomfunktion gebildet und konfiguriert ist, um die Parameter des Anti-Müller-Hormons (AMH), des follikelstimulierenden Hormons (FSH), Estradiols (E2), der Antralfollikelzählung (AFC), des Flussindex (FI), des Vaskularisationsindex (VI), des vaskulären Flussindex (VFI) und des Ovarialvolumens zu kombinieren;
- Bereitstellen, als einen Ausgangswert des Polynomalgorithmus, des Eierstockalters einer Frau als eine Funktion der Bluthormon- und transvaginalen Ultraschallwerte;
- Vergleichen des Eierstockalterwerts der Frau mit dem Wert des Frauenalters;

wobei der Schritt des Bereitstellens des Eierstockalters der Frau als eine Funktion der Bluthormon- und transvaginalen Ultraschallwerte als einen Ausgangswert des Polynomalgorithmus die Verwendung der folgenden Gleichung umfasst:

$$\texttt{Eierstockalter = c + a*AMH + b*bFSH + c*E2 + d*AFC +}$$

$$\texttt{e*FI + f*VFI + g*Volumen.}$$

**3.** Ultraschall-Diagnosevorrichtung für die Verarbeitung von klinischen und biochemischen Daten und dreidimensionalen (3D) Ultraschallbildern zur Vorhersage des Eierstockalters einer Frau, **dadurch gekennzeichnet, dass** sie die Vorrichtung gemäß Anspruch 1 umfasst.

**4.** Verarbeitungsprogramm zur Verarbeitung von klinischen und biochemischen Daten und dreidimensionalen (3D) Ultraschallbildern zur Vorhersage des Eierstockalters einer Frau, **gekennzeichnet durch** die Anwendung des Verfahrens nach Anspruch 2 auf einer "webbasierten" Software, die in der Lage ist, das Eierstockalter einer Frau in Echtzeit bereitzustellen.

**Revendications**

**1.** Un appareil (100) pour le traitement des données cliniques et biochimiques et des échographies tridimensionnelles (3D) pour la prédiction de l'âge ovarien de la femme, comprenant :

- au moins un bloc fonctionnel (101) configuré pour mesurer les valeurs de l'échographie transvaginale du volume ovarien, du nombre de follicules antraux (AFC), de l'indice de vascularisation (VI), de l'indice de fluidité (FI), de l'indice de flux sanguin (VFI) d'au moins une femme ;
- et au moins un bloc fonctionnel (102) configuré pour mesurer les valeurs hormonales sanguines d'au moins une femme faisant partie du groupe constitué par l'hormone anti-mullérienne (AMH), l'hormone folliculo-stimulante (FSH), l'oestradiol (E2), chez les femmes en bonne santé pendant une période de temps comprise entre le premier et le quatrième jour du cycle menstruel ;

les signaux de sortie de ces blocs (101, 102) étant les signaux d'entrée d'un bloc de traitement des données (103) sur la base d'un modèle linéaire généralisé (MLG) constitué par une fonction polynomiale configurée pour :

- combiner les paramètres de l'hormone anti-mullérienne (AMH), de l'hormone folliculo-stimulante (FSH), de l'oestradiol (E2), du nombre de follicules antraux (AFC), de l'indice de fluidité (FI), de l'indice de vascularisation (VI), de l'indice de flux sanguin (VFI) et du volume ovarien, et
- fournir comme résultat de sortie de l'algorithme polynomial la valeur de l'âge ovarien en fonction des valeurs hormonales sanguines et des valeurs de l'échographie transvaginale, évaluée en années et comparée à l'âge de la femme ;
où la fonction polynomiale correspond à l'équation :

$$\texttt{âge ovarien = c + a*AMH + b*bFSH +c*E2 + d*AFC + e*FI}$$

$$\texttt{+ f*VFI + g*Volume.}$$

**2.** Une méthode de traitement des données cliniques et biochimiques et des échographies tridimensionnelles (3D) pour la prédiction de l'âge ovarien de la femme, comprenant les étapes suivantes :

- Mesurer, par une simple analyse sanguine, les valeurs hormonales sanguines d'au moins une femme faisant partie du groupe constitué par l'hormone anti-mullérienne (AMH), l'hormone folliculo-stimulante (FSH), l'oestradiol (E2), chez les femmes en bonne santé pendant une période de temps comprise entre le premier et le quatrième jour du cycle menstruel ;

- Mesurer, par une échographie 3D, les valeurs de l'échographie transvaginale du volume ovarien, du nombre de follicules antraux (AFC), de l'indice de vascularisation (VI), de l'indice de fluidité (FI), de l'indice de flux sanguin (VFI) chez au moins une femme ;
- Effectuer le traitement des données des valeurs hormonales sanguines et des valeurs de l'échographie transvaginale au moyen d'un modèle linéaire généralisé (MLG) de traitement constitué par une fonction polynomiale configurée pour combiner les paramètres de l'hormone anti-mullérienne (AMH), de l'hormone folliculo-stimulante (FSH), de l'oestradiol (E2), du nombre de follicules antraux (AFC), de l'indice de fluidité (FI), de l'indice de vascularisation (VI), de l'indice de flux sanguin (VFI) et du volume ovarien ;
- Fournir, en tant que valeur de sortie de l'algorithme polynomial, l'âge ovarien de la femme en fonction des valeurs hormonales sanguines et des valeurs de l'échographie transvaginale ;
- Comparer la valeur de l'âge ovarien avec la valeur de l'âge de la femme ;

où l'étape consistant à fournir, en tant que valeur de sortie de l'algorithme polynomial, l'âge ovarien de la femme en fonction des valeurs hormonales sanguines et des valeurs de l'échographie transvaginale comprend l'utilisation de l'équation :

```
âge ovarien = c + a*AMH + b*bFSH +c*E2 + d*AFC + e*FI
+ f*VFI + g*Volume.
```

3. Un appareil de diagnostic par ultrasons pour le traitement des données cliniques et biochimiques et des échographies tridimensionnelles (3D) pour la prédiction de l'âge ovarien de la femme correspondant, **caractérisé en ce qu'**il comprend l'appareil selon la revendication 1.

4. Un programme de traitement pour le traitement des données cliniques et biochimiques et des échographies tridimensionnelles (3D) pour la prédiction de l'âge ovarien de la femme, **caractérisé par** la mise en oeuvre de la méthode selon la revendication 2 sur un logiciel « accessible sur le Web » capable de fournir l'âge ovarien de la femme en temps réel.

100

102 — Blood Hormone Levels Values Measuring

101 — Transvaginal Sonographic Values Measuring

103 — Processing Values By means of a Generalized Linear Model (GLM)

→ Ovarian age

FIG. 1

Transvaginal
Utrasound
Values
Measuring

New Patient

Blood
Hormonal Values
Measuring

Patient Values
Processing

Ovarian Age

Ovarian Age < = > Chronological Age

FIG. 2

FIG. 3

FIG. 4

EP 3 048 981 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MARIA ELISABETTA COCCIA et al.** Ovarian reserve. *ANNALS OF THE NEW YORK ACADEMY OF SCIENCES,* 01 April 2008, vol. 1127 (1), 27-30
- Establishing the intercycle variability of three-dimensional ultrasonographic predictors of ovarian reserve. **JAYAPRAKASAN K et al.** FERTILITY AND STERILITY. ELSEVIER SCIENCE INC, 01 December 2008, vol. 90, 2126-2132

- A simple multivariate score could predict ovarian reserve, as well as pregnancy rate, in infertile women. **YOUNIS J S et al.** FERTILITY AND STERILITY. ELSEVIER SCIENCE INC, 01 July 2010, vol. 94, 655-661